# EUROPÄISCHE PATENTSCHRIFT

(11) **EP 2 699 901 B1**
(45) Veröffentlichungstag und Bekanntmachung des Hinweises auf die Patenterteilung: **23.08.2017**
(21) Anmeldenummer: 13709384.5
(22) Anmeldetag: 05.03.2013
(51) Int. Cl.: G01N 33/38

(54) **FEUCHTEMESSUNG FÜR FUSSBÖDEN AUS BETON**
MOISTURE MEASUREMENT FOR CONCRETE FLOORS
MESURE DE L'HUMIDITÉ DE SOLS CONSTITUÉS DE BÉTON

(30) Priorität: 14.05.2012 DE 102012208050
(43) Veröffentlichungstag der Anmeldung: 26.02.2014
(73) Patentinhaber: Geretshauser, Alfred, 85229 Markt Indersdorf (DE); Zehrer, Sven, 85229 Markt Indersdorf (DE)
(72) Erfinder: Geretshauser, Alfred, 85229 Markt Indersdorf (DE); Zehrer, Sven, 85229 Markt Indersdorf (DE)
(74) Vertreter: Jöstingmeier, Martin
(86) Internationale Anmeldenummer: PCT/EP2013/054444
(87) Internationale Veröffentlichungsnummer: WO 2013/170973

(56) Entgegenhaltungen:
- WO-A1-2005/053350
- DE-A1-102010 043 083
- CHIH-YUAN CHANG ET AL: "Implementing RFIC and sensor technology to measure temperature and humidity inside concrete structures", CONSTRUCTION AND BUILDING MATERIALS, ELSEVIER, NETHERLANDS, Bd. 26, Nr. 1, 18. Juni 2011 (2011-06-18), Seiten 628-637, XP028285575, ISSN: 0950-0618, DOI: 10.1016/J.CONBUILDMAT.2011.06.066 [gefunden am 2011-06-27]
- NORRIS ET AL: "Temperature and moisture monitoring in concrete structures using embedded nanotechnology/microelectromechanical systems (MEMS) sensors", CONSTRUCTION AND BUILDING MATERIALS, ELSEVIER, NETHERLANDS, Bd. 22, Nr. 2, 9. November 2007 (2007-11-09), Seiten 111-120, XP022338775, ISSN: 0950-0618, DOI: 10.1016/J.CONBUILDMAT.2006.05.047
- Halil Ceylan ET AL: "A Feasibility Study on Embedded Micro-Electromechanical Sensors and Systems (MEMS) for Monitoring Highway Structures - IHRB Project TR-575", , Juni 2011 (2011-06), XP055063247, Gefunden im Internet: URL:http://www.intrans.iastate.edu/reports /tr-575_ceylan_mems_w_cvr.pdf [gefunden am 2013-05-16]
- "Raychem Freiflächenbeheizung für Fahrbahnen, Rampen und Fußwege", , 2010, XP055063380, 69126 Heidelberg, Germany Gefunden im Internet: URL:http://www.kambach-gmbh.de/Pdf/raychem /Freiflaechen.pdf [gefunden am 2013-05-17]
- "Gerätebeschreibung Arnold Feuchtemesssonden Typ FS(x)", , 18. Januar 2007 (2007-01-18), XP055063249, D-79793 Wutöschingen-Degernau, Germany Gefunden im Internet: URL:http://www.werne-thiel.de/pdf/download 8.pdf [gefunden am 2013-05-16]

## Beschreibung

### Technisches Gebiet

Die Erfindung betrifft die Bestimmung der Restfeuchte eines Estrichs und ein dafür geeignetes Messgerät.

### Stand der Technik

Fußböden bestehen in der Regel aus wenigstens drei Schichten, denen verschiedene Funktionen zukommen: Auf einer Tragschicht, z.B. einer Geschossdecke oder einer Bodenplatte wird zunächst Estrich aufgebracht, auf den wiederum ein Fußbodenbelag aufgebracht wird. Der Estrich befindet sich somit zwischen der tragenden Geschoßdecke, bzw. Bodenplatte, und dem Fußbodenbelag. Manchmal werden Schall und/oder Wärmeisolationsschichten zwischen der Tragschicht und dem Estrich eingebracht. Meist wird Zementestrich verwendet, der unter kontrollierten Bedingungen aushärten muss, bis eine Restfeuchte vom maximal 2,0 CM% bei unbeheiztem Estrich, bzw. 1,8 CM% bei beheiztem Estrich unterschritten wird. Das gilt entsprechend für bei Anhydritestrich; hier gelten geringere Grenzwerte für die zulässige Restfeuchte (unbeheizt: 0,5 CM%; beheizt: 0,3 CM%). Oft werden Estriche verwendet, denen Zusätze zugegeben wurde, damit sie schneller aushärten, man spricht dann von beschleunigen Estrichen. Auch bei diesen beschleunigten Estrichen ist eine Kontrolle der Restfeuchte wichtig. Wird ein Fußbodenbelag auf einen nicht ausreichend trockenen Estrich aufgelegt, besteht die Gefahr von Bauschäden.

Zur Bestimmung der Restfeuchte wird in der Regel die Kalzium-Karbid-Methode verwendet. Dazu wird dem Estrich eine Probe entnommen, gewogen, in einen Druckbehälter eingebracht und darin im Beisein von Kalzium-Karbid zerkleinert. Das in der Probe enthaltende Wasser reagiert zu Acetylen. Der entsprechende Druckanstieg dient als Maß für die Menge des in der Probe enthaltenen Wassers.

Diese Methode erlaubt keine zerstörungsfreie Messung der Restfeuchte und ist anfällig für Messfehler.

Aus der EP 1 817 529 B1 ist ein Sensor zur Erfassung von Messwerten wie Temperatur oder Feuchtigkeit bekannt. Der Sensor dient zur Überwachung von Holzfußbodenbelägen, wie beispielsweise Parkettböden und wird auf dem Estrich in einer Ausnehmung an der Unterseite des Holzfußbodenbelags angeordnet. Die erfassten Messwerte werden gespeichert und können über eine RFID-Datenstrecke ausgelesen werden.

Aus der DE 103 55 368 A1 ist ein Messgerät zur Bestimmung der Restfeuchte eines Estrichs bekannt. Dass Gerät hat ein Gehäuse, in dem eine Messelektronik angeordnet ist. Das Gehäuse wird bündig mit der Oberkante in den feuchten Estrich eingesetzt wird und hat Schlitze, über die Feuchtigkeit aus dem Estrich in das Gehäuse diffundieren kann, um über die Luftfeuchte in dem Gehäuse auf die Restfeuchte des Estrichs zu schließen. Nachdem die Messung abgeschlossen ist, kann die Messelektronik durch die Öffnung aus dem Gehäuse entfernt werden. Das Gehäuse verbleibt im Estrich.

DE 10 2005 017 550 A1 offenbart ein Messgerät zu Feuchtemessung mit einer Messkammer, die auf einen Prüfkörper, z.B. einen Estrich, gasdicht aufgesetzt wird. Zur Messung wird in der Messkammer ein Unterdruck erzeugt und dabei wird die Luftfeuchte in der Messkammer gemessen. Problematisch ist jedoch, dass die Verdunstungsrate vom Estrich in die Messkammer und damit die Luftfeuchte durch den Unterdruck erhöht wird. Dadurch wird dem Estrich im Bereich unter der Messkammer freies Wasser entzogen, dass aus dem übrigen Estrich bis zu nächsten Messung nachdiffundieren muss, ansonsten ergibt die zweite Messung eine zu geringe Restfeuchte. Dieses Nachdiffundieren erfolgt aber mit zunehmender Trockung und damit verbundener Aushärtung des Estrichs immer langsamer. Eine kontinuierliche Messung ist daher nicht möglich.

Construction and Building Materials, Bd. 26, Seiten 628-637 (2012) ist ein Bericht über eine Studie zur Entwickung eines Meßgeräts zur Messung der Feuchte und der Temperatur von Beton, um mögliche Korrosionsschäden der Armierung besser Abschätzen zu können. Dazu wurde unter anderem ein Gehäuse für ein Messgerät entwickelt, dass in die tragenden Strukturen eines Gebäudes einbetoniert wird. Ein Teil des Gehäuses ist selbst aus Beton wodurch bei hoher Widerstandkraft eine optimale Anbindung an den späteren Einsatzort gegeben ist.
Construction and Building Materials, Bd. 22, Seiten 111-120, (2007) beschreibt die Entwicklung eines mikro-elektromechanischen Sensors zur Messung der relativen Feuchtigkeit sowie der Temperatur. Der Sensor hat Messstreifen, die auf einer Seite mit einem wasserdampfempfindlichen Polymer beschichtet ist. Die Länge der Polymerbeschichtung verändert sich als Funktion der Umgebungsfeuchte, was zu einer messbaren Scherung des Messstreifens führt.
*A Feasibility Study on Embedded Micro-Electromechanical Sensors and Systems (MEMS) for Monitoring Highway Structures* des National Concrete Pavement Technology Center, (Final Report June 2011) ist eine Machbarkeitsstudie, die zunächst die Entwicklung der MEMS, ihre Anwendung im Bereich des Straßenbaus sowie die Vernetzung von Sensoren mit Auswerteeinheiten beschreibt. Dem schließt sich ein Ausblick in die Zukunft an Es werden zudem Versuche mit am Markt befindlichen Sensoren beschrieben. Dazu wurden Röhren in einen Betonkubus einbetoniert, in die Sensoren eingebracht wurden.
Raychem *Freiflächenbeheizung der Fahrbahn, Rampen und Fußwege* ist eine Produktbeschreibung eines Heizsystems für Rampentreppen und Gehwege. Dabei wird ein Bodentemperatur und Feuchtesensor "VIA-DU-S20" verwendet. Dieser Sensor wird jedoch nach den Skizzen auf S. 31 bündig mit der Oberseite eines Zementestrichs angeordnet, eine Befestigung an einer Tragschicht ist nicht offenbart.

*Gerätebeschreibung Arnold Feuchtemesssonden Typ FS(x)* beschreibt eine Vielzahl von Feuchtesensoren, die beispielsweise zur Bestimmung der Getreidefeuchte in einem Silo, von Schüttgut auf einem Förderband etc. Dazu werden die Sensoren über Ausleger an feststehenden Strukturen frei hängend befestigt.

### Darstellung der Erfindung

Die Erfindung beruht auf der Beobachtung, dass in der Praxis zur Messung der Restfeuchte von Estrich in der Regel eine Probe entnommen wird.

Der Erfindung liegt die Aufgabe zugrunde, die Restfeuchte eines Estrichs zu bestimmen. Diese Aufgabe wird durch einen Estrich mit Messgerät nach Anspruch 1 gelöst. Vorteilhafte Ausgestaltungen der Erfindung sind in den Unteransprüchen angegeben.
Das Messgerät dient zur Bestimmung von Parametern von Estrichen und kann im Estrich angeordnet werden. Dazu wird das Messgerät beim Verlegen des Estrichs in den noch nicht abgebundenen Estrich eingelegt. Beispielsweise kann das Messgerät auf die Schicht auf die der Estrich aufgebracht wird, aufgelegt werden. Vorzugsweise kann es dort fixiert werden. Anschließend wird der Estrich wie üblich verlegt.
Vorzugsweise wird vor dem Verlegen des Estrichs ein nach oben weisendes Hinweismittel, z.B. ein Fähnchen oder ein vorzugsweise flexibler Stab an dem Messgerät, vorzugsweise an seinem Gehäuse, befestigt. Dieses Hinweismittel zeigt die Position des Messgeräts an, wenn es vom Estrich ansonsten vollständig bedeckt ist. Dadurch kann es später leicht im Estrich lokalisiert werden, z.B. um ein Lesegerät zum Auslesen von Messdaten über eine Funkdatenstrecke in die Nähe des Messgeräts bringen zu können. Zudem zeigt das Hinweismittel beim Verlegen des Estrichs die Position des Messgeräts an, so dass die Estrichleger in diesem Bereich entsprechen vorsichtig arbeiten um das Messgerät nicht zu beschädigen. Nachdem die Restfeuchte einen vorgegebenen Wert unterschritten hat und der Estrich somit belegreif ist, kann das Hinweismittel einfach bündig mit der Estrichoberseite gekappt werden. Anschließend kann die nächste Schicht aufgebracht werden.

Das Messgerät, zumindest sein Gehäuse wird vollständig im Estrich eingeschlossen. Umgebungsparameter, wie die Luftfeuchte oberhalb des Estrichs können die Messung dann nicht beeinflussen. Auch eine Beschädigung des Messgeräts z.B. durch herunterfallende Werkzeuge, Leitern etc. ist ausgeschlossen, weil das Messgerät vollständig im Estrich eingeschlossen ist. Das Messgerät hat ein Gehäuse, in dem wenigstens ein Feuchtesensor angeordnet ist. Das Gehäuse kann wenigstens einen freien Innenraum aufweisen, der über wenigstens eine, bevorzugt wenigstens zwei wasserdampfdurchlässige Öffnungen mit der Umgebung des Gehäuses kommuniziert, d.h. es ist zumindest ein Austausch von Wasserdampf zwischen dem Estrich und dem als Messkammer dienenden Innenraum gegeben. Deshalb stellt sich ein Gleichgewicht zwischen der Luftfeuchte in dem Innenraum und dem Estrich ein. Vorzugsweise ist der Feuchtesensor in dem Innenraum angeordnet. Wird solch ein Messgerät beim Aufbringen des Estrichs auf eine Geschoßdecke bzw. Bodenplatte in den Estrich eingebracht, dann stellt sich beim Aushärten des Estrichs in dem Innenraum eine Luftfeuchte ein, die ausschließlich von der Restfeuchte des Estrichs und der Temperatur (streng genommen auch vom Druck) in dem freien Innenraum abhängt. Die Luftfeuchte im Innenraum kann mittels des Feuchtsensors als Maß für die Restfeuchte des Estrichs bestimmt werden. Der freie Innenraum bildet dann eine Messkammer. Dazu können bekannte und günstig verfügbare Luftfeuchtesensoren verwendet werden. Die in der Praxis nicht zerstörungsfreie direkte Messung der Restfeuchte wird somit durch eine indirekte Messung ersetzt, wobei der Innenraum die Funktion einer Messkammer für den (Luft-)Feuchtesensor hat.
Das Messgerät kann im Estrich selbst angeordnet werden. Bevorzugt ist das Messgerät vollständig im Estrich eingeschlossen, und deshalb von außen nicht sichtbar. Zudem ist so sichergestellt, dass die Feuchte oder sonstige Parameter im Innenraum nur durch den Estrich und nicht unmittelbar durch die Umgebung oberhalb des Estrichs bestimmt werden. Um sicherzustellen, dass das Messgerät vollständig im Estrich eingeschlossen wird, hat es Befestigungsmittel zu Fixierung des Messgeräts an der unter dem Estrich angeordneten Schicht, nachfolgend kurz Tragsschicht (auch wenn die Schicht eine andere Funktion hat, z.B. Schall und/oder Wärmeisolation). Durch diese Befestigungsmittel kann das Messgerät an einer gegebenen Stelle auf der Tragschicht fixiert werden. Es wird somit ausgeschlossen, dass das Messgerät beim Verlegen des Estrichs seine Position verändert. Die spätere Kenntnis der Position ist insbesondere dann vorteilhaft, wenn vom Messgerät gesammelte Daten über eine Nahfeld Funkdatenstrecke mittels eines entsprechende Lesegeräts abgefragt werden sollen, denn dann muss zumindest dessen Antenne in die Nähe des Messgeräts gebracht werden. Zudem kann über die Befestigungsmittel der Abstand des Messgeräts von der Tragschicht und auch zur Oberkante des fertigen Estrichs eingestellt werden. Vorzugsweise wird durch die Befestigungsmittel das Messgerät zumindest in etwa in der Mitte des Estrichs (+-25%) angeordnet. Weil die Dicke des aufzubringenden Estrichs von Baustelle zu Baustelle variieren kann, haben die Befestigungsmittel vorzugsweise eine Verstellmöglichkeit. Dadurch kann der Abstand des Messgeräts von der Tragsschicht und damit seine Lage im Estrich eingestellt werden. Zusätzlich kann das schon beschriebene Hinweismittel am Gehäuse des Messgeräts angeordnet sein.
Die wenigstens eine Öffnung des Gehäuses ist bevorzugt durch eine gas-, insbesondere wasserdampfdurchlässige semipermeable Membran verschlossen. Die Membran kann z.B. eine entsprechende Textilmembran sein. Solche semipermeable Membranen ermöglichen eine besonders schnelle und zuverlässige Messung, weil dadurch ausgeschlossen werden kann, dass flüssiges Wasser in den Innenraum gelangt, wodurch die Messung verfälscht würde. Sofern die Öffnung(en) nicht durch eine semipermeable Membran verschlossen sind, sollte nach dem Einbringen des Messgeräts in den Estrich abgewartet werden, bis der Estrich eventuell in den Innenraum eingedrungenes Wasser absorbiert hat. Dazu kann der Innenraum bevorzugt an seiner tiefsten Stelle eine Ablauföffnung aufweisen.

Bevorzugt wird die Membran durch ein außen vor der Membran angeordnetes Gitter oder dgl. geschützt. Natürlich kann die Öffnung, bzw. können die Öffnungen auch ohne dass sie durch eine Membran verschlossen sind durch ein Gitter geschützt werden. Das Gitter kann in das Gehäuse integriert sein, insbesondere einstückig mit einem Gehäuseteil ausgeführt sein.

Vorzugsweise hat der Innenraum zwei dampfdurchlässige Öffnungen. Beispielsweise kann der Innenraum als Kanal ausgebildet sein, der die beiden Öffnungen miteinander verbindet. In Versuchen hat sich gezeigt, dass ein in einem solchen Kanal angeordneter Feuchtesensor Änderungen besonders schnell und zuverlässig detektiert.

Bevorzugt liegt wenigstens eine erste Öffnung über wenigstens einer zweiten Öffnung. Dadurch wird die Messung genauer, denn Estrich hat beim Aushärten einen ausgeprägten Feuchtegradienten, weil die Restfeuchte nach oben hin ab nimmt. Durch diesen Feuchtegradienten entsteht zwischen den beiden Öffnungen eine gerichtet Strömung der Wassermoleküle durch den Innenraum. Dadurch entsteht in dem Innenraum eine für die mittlere Feuchte repräsentative Luftfeuchte, die dann gemessen wird. Zudem wird durch die Strömung der Austausch der Gasmoleküle in dem Innenraum verbessert. Auch dies erhöht die Genauigkeit.

Beispielsweise kann das Gehäuse zusätzlich zu dem als Messkammer dienenden Innenraum ein zumindest im Wesentlichen geschlossenes Abteil aufweisen, in dem eine Mess- und/oder Kommunikationsschaltung angeordnet ist, welche mit dem Feuchtesensor verbunden ist. Die Mess- und/oder Kommunikationsschaltung ermöglicht eine Erfassung und Weiterleitung von Messwerten, die durch Sensoren, z.B. einen oder mehrere Feuchtsensoren, einen oder mehrere Temperatursensoren gemessen werden.

Zur Weiterleitung von Messwerten kann das Messgerät z.B. mit einem Lesegerät über eine bevorzugt bidirektionale Datenstrecke kommunizieren. Die Kommunikation kann vorzugsweise über eine Funkdatenstrecke, z.B. mittels der üblichen RFID-Verfahren, und/oder über Kabel und/oder Lichtleiter erfolgen.

Das Abteil mit der Mess- und/oder Kommunikationsschaltung ist vorzugsweise vergossen. Dadurch wird die Mess- und/oder Kommunikationsschaltung zuverlässig vor eindringender Feuchtigkeit geschützt.

Das Messgerät hat vorzugsweise weitere Sensoren, wie z.B. wenigstens einen Dehnungsmessstreifen und/oder Drucksensor zur Bestimmung von vom Estrich auf das Gehäuse übertragener Kraft bzw. Druck. Dadurch lassen sich mechanische Belastungen des Estrichs und auch Schwingungen wie Trittschall messen. Die entsprechenden Messwerte können z.B. durch die Mess- und/oder Kommunikationsschaltung an eine Zentrale einer Raum- und/oder Gebäudeüberwachung, wie z.B. einer Alarmanlage, übermittelt werden. Auch seismische Erschütterungen können so bestimmt und bei einem Überschreiten eines vorgegebenen Wertes kann ein Alarm ausgelöst werden. Dazu kann das Messgerät über eine Datenstrecke mit einer Alarmzentrale und/oder einer Gebäudesteuerung verbunden sein.

Bevorzugt hat das Messgerät wenigstens einen Temperaturfühler, der vorzugsweise mit der Mess- und/oder Kommunikationsschaltung verbunden ist. Die Temperatur ist eine wichtige Kenngröße bei der Bewertung der gemessenen Luftfeuchte. Wenn eine Fußbodenheizung im Estrich verlegt ist, sollte zum Aushärten des Estrichs eine vorgegebene Temperaturkurve abgefahren werden. Mittels des Temperatursensors kann die tatsächliche Temperaturkurve gemessen werden. Die gemessene Temperaturkurve wird vorzugsweise gespeichert, z.B. in der Mess- und/oder Kommunikationsschaltung und kann bei Mängeln am Estrich später ausgelesen und mit der vorgegebenen Temperaturkurve verglichen werden, um eine mögliche Ursache für den Mangel einzugrenzen. Besonders bevorzugt kommuniziert das Messgerät mit der Steuerung der Fußbodenheizung, um z.B. die aktuell gemessene Temperatur an die Steuerung zu übermitteln. Entsprechend kann die Steuerung die Heizleistung in Abhängigkeit von der gemessenen Temperatur einstellen, also regeln oder steuern.

Die Erfindung betrifft zudem ein Verfahren zur Kontrolle der Belegreife eines Estrichs. Als Belegreife wird der Zustand bzw. der Zeitpunkt verstanden, wenn der Estrich ausreichend ausgehärtet und dann auch ausreichend ausgetrocknet ist, um den eigentlichen Fußbodenbelag, z.B. ein Holzparkett, aufbringen zu können. Ist der Estrich noch nicht ausreichend trocken, wird z.B. durch Klebstoffe mittels denen der Fußbodenbelag auf den Estrich geklebt wird, oftmals zumindest ein Teil des Estrichs aufgeweicht. Zudem neigt ein auf einem nicht belegreifen Estrich aufgebrachter Holzfußboden zu Schimmelbildung sowie zur Verwerfungen. Deshalb ist es wichtig die Belegreife eines Estrichs festzustellen. Dazu wird bevorzugt die Restfeuchte des Estrichs als Funktion der Zeit erfasst. Die Belegreife kann als der Zeitpunkt erfasst werden, an dem die Restfeuchte einen vorgegebenen Wert unterschreitet. Alternativ oder zusätzlich kann man den Zeitpunkt der Belegreife auch dadurch bestimmen, dass die Restfeuchte gegen einen Wert konvergiert ist. Letztere Variante hat den Vorteil, dass eine Eichung des Feuchtesensors nicht notwendig ist.

Die dazu notwendige Messung der Restfeuchte erfolgt bevorzugt indirekt über die Messung der Feuchte eines Gases, z.B. Luft, in einem im Estrich eingeschlossenen Hohlraum, der wasserdampfdiffusionsoffen mit dem Estrich verbunden ist.

Die Restfeuchte gilt als konvergiert, wenn die Änderung der Restfeuchte (bei vorzugsweise zumindest in etwa konstanter Temperatur) zwischen wenigstens zwei beispielsweise aufeinanderfolgenden Messung einen vorgegebenen Wert unterschreitet. Insbesondere wenn die Messungen der Restfeuchte nicht in konstanten Intervallen durchgeführt werden sollte die Steigung der Restfeuchte als Funktion der Zeit, welche durch Bildung des sogenannten Differenzenquotienten einfach genähert werden kann, einen vorgegebenen Wert unterschreiten.

Das Erfassen und Messen der Restfeuchte als Funktion der Zeit kann mittels des oben beschriebenen Messgeräts erfolgen. Man kann solch ein Messgerät auch als Restfeuchtesensor bezeichnen. Das Erfassen der Restfeuchte als Funktion der Zeit erfolgt vorzugsweise in Intervallen. Dadurch kann der Energiebedarf des entsprechenden Messgeräts minimiert werden. Bei einem batterieversorgten Messgerät steigt somit die Dauer während der Estrichparameter, also Messwerte im Estrich erfasst werden können. Zudem wird der benötigte Speicher zur Aufzeichnung der Messwerte reduziert. Beispielsweise können die Intervalle zwischen zwei Feuchtemessungen als Funktion der Veränderung der Feuchte und/oder der Temperatur zwischen wenigstens zwei bevorzugt aufeinanderfolgenden Messungen bestimmt werden. Es sollte ein maximales Intervall, also eine maximale Zeitspanne zwischen zwei Messungen vorgegeben sein.

Vorzugsweise wird die Luftfeuchte in der Messkammer und somit die Restfeuchte des Estrichs (und bevorzugt der Temperatur) regelmäßig erfasst, z.B. in stündlichen Intervallen. Die Aufzeichnung, also Speicherung der Messdaten erfolgt vorzugsweise erst bei einem Absinken der Luftfeuchte unter einen ersten vorbestimmten Wert von z.B. 98%, da erst bei einem Wert kleiner als 100% eine Trocknung messbar ist. Anschließend erfolgt eine regelmäßige Speicherung der gemessenen Werte, wobei zwischen zwei gespeicherten Werten eine erste gegebene Zeit t₁ verstreicht. Beim Auslesen der gespeicherten Werte lässt sich somit eine die Restfeuchte als Funktion der Zeit auftragen und Zwischenwerte können durch Interpolation ermittelt werden. Wenn die gemessene Luftfeuchte und damit die Restfeuchte unter einen zweiten vorbestimmten Wert sinkt, dann kann vorzugsweise zwischen zwei aufeinanderfolgend gespeicherten Werten eine zweite gegebene Zeit t₂ verstreichen, wobei t₂ größer als t₁ ist; beispielsweise kann t₁ 1h (sinnvoll 20min bis 4h) betragen und t₂ kann 1h bis 12h betragen. Zusätzlich können vorzugsweise Zwischenwerte gespeichert werden, wenn die Temperatur als Funktion der Zeit einen Gradienten aufweist, der größer ein gegebener Gradient ist. Zudem sollte vorzugsweise die gemessene Maximaltemperatur gespeichert werden.

Bevorzugt wird die Temperatur des Estrichs als Funktion der Zeit gemessen und erfasst. Auch hierzu wird vorzugsweise wieder in Intervallen gemessen. Treten später Schäden im Estrich auf, können diese Daten ausgelesen werden. Dadurch kann ex post überprüft werden, ob z.B. eine Ausheizkurve zum Trocknen und Aushärten des Estrichs vorschriftsgemäß abgefahren wurde. Wenn das Messgerät die gemessene Temperatur an eine Steuerung für die Heizung übermittelt, kann die Ausheizkurve als Funktion der Estrichtemperatur gesteuert und/oder geregelt werden. Die Estrichtemperatur sollte besonders, wenn der Estrich mit Holz belegt wurde, eine vorgegebene Temperatur nicht übersteigen, um Schäden am Holz zu vermeiden. Auch dies kann durch Abfragen der zuvor erfassten Temperaturwerte kontrolliert werden und im Fall, dass das Messgerät zumindest die gemessene Temperatur an eine Heizungssteuerung überträgt auch durch entsprechende Regelung und/oder Steuerung verhindert werden.

Die gemessene Temperatur als Funktion der Zeit kann mit einem gegebenen Temperaturverlauf verglichen werden. Anhand des Vergleichs kann bestimmt werden, ob der Estrich belegreif ist.

Die Erfindung betrifft neben dem Messgerät auch einen Fußboden mit einem Estrich in dem ein Messgerät nach der Erfindung vorzugsweise vollständig aufgenommen ist. Die Oberseite des Messgeräts ist dann vorzugsweise vollständig mit Estrich bedeckt. In gleicher Weise kann das Messgerät auch in andere beim Aushärten trocknende Bauelemente, z.B. Betonwände eingebracht werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt ein Messgerät im Schnitt, und
Figur2 zeigt ein weiteres Messgerät im Schnitt.

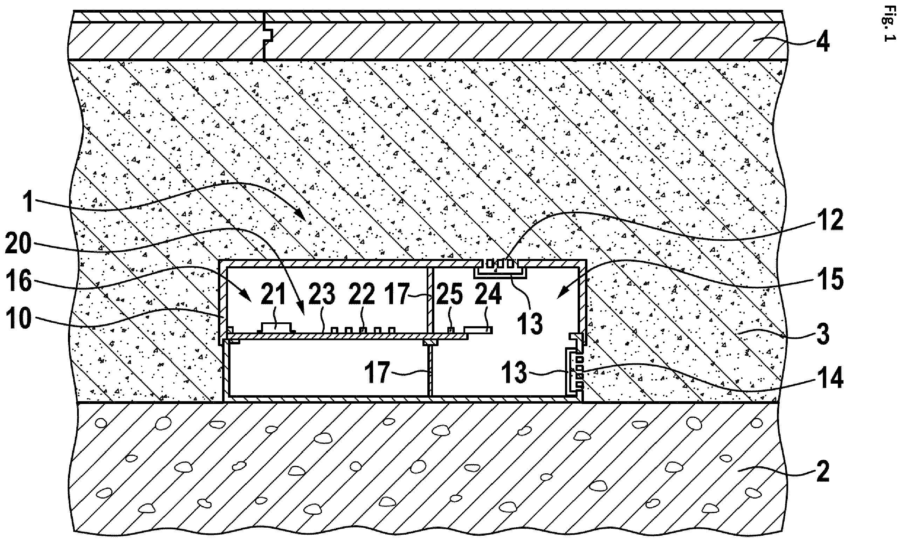

In Figur 1 ist ein Messgerät 1 zur Bestimmung der Restfeuchte von Estrich in einer typischen Einbausituation dargestellt. Das Messgerät 1 hat näherungsweise die Form einer flachen Schachtel, und liegt auf der Oberseite einer Tragschicht 2, die z.B. eine Geschossdecke oder eine Bodenplatte sein kann auf. Auf der Tragschicht 2 wurde ein Estrich 3 verlegt, so dass das Messgerät 1 im Estrich eingeschlossen, sozusagen "eingegossen" ist. Lediglich zur Anschauung ist ein auf dem Estrich verlegter Holzfußboden 4 dargestellt.

Das Messgerät 10 hat ein hier beispielhaft zweiteiliges Gehäuse 10 mit einem Innenraum, der von zwei Stegen 17 in ein Abteil 16 und eine Messkammer 15 unterteilt ist. In der Kammer 16 ist eine Mess- und Kommunikationsschaltung 20 angeordnet. Bevorzugt ist die Kammer mit einer Vergussmasse ausgefüllt, d.h. die Mess- und Kommunikationsschaltung 20 ist mit der Vergussmasse vergossen und so dauerhaft vor Feuchtigkeit und mechanischer Beanspruchung geschützt.

Das Messgerät 10 kann mit wenigstens einem Befestigungsmittel vor dem Verlegen des Estrichs an der Tragschicht 1 befestigt sein. Das Befestigungsmittel kann beispielsweise wenigsten eine an das Gehäuse 10 angesetze Lasche sein, durch die das Messgerät 10 mittels wenigstens einer Schraube einem Nagel oder einem anderem Verbindungsmittel an der Tragschicht fixiert werden kann.

Die Messkammer hat vorzugsweise zwei übereinander angeordnete Öffnungen 12, 14. Die Öffnungen 12, 14 sind vorzugsweise gitterartig ausgeführt, so dass keine Feststoffe des zunächst flüssigen Estrichs in das Gehäuse eindringen können. Um zu verhindern, dass flüssiges Wasser durch die Öffnungen in den Innenraum gelangt, können die beiden Öffnungen 12, 14 je durch eine semipermeable dampfdurchlässige Membran 13 verschlossen sein. Die semipermeablen Membranen 13 sind bevorzugt an der Innenseite des Gehäuses vor den jeweiligen Öffnungen 12, 14 angeordnet. Zur Messung der Feuchte sollte die Messkammer dampfdurchlässig mit dem Estrich verbunden ist, so dass sich ein Gleichgewicht zwischen der Luftfeuchte in der Messkammer und der Restfeuchte des Estrichs einstellt. Dadurch kann anhand einer Messung der Luftfeuchte in der Messkammer auf die Restfeuchte des Estrichs geschlossen werden. Folglich ist in der Messkammer ein Feuchtesensor 24 angeordnet. In der gezeigten bevorzugten Ausführungsform hat das Gehäuse 10 zwei übereinander angeordnete dampfdiffusionsoffene Öffnungen 12, 14. Der untere Bereich des Estrichs trocknet in der Regel langsamer aus, weil trocknende Luft nur über die Oberfläche des zu diesem Zeitpunkt noch unbelegten Estrichs streicht. Deshalb ist der Wasserdampfpartialdruck im Bereich vor der unteren Öffnung 14 größer als im Bereich vor der oberen Öffnung 12. Folglich kommt es zu einer gerichteten Diffusion durch die Messkammer. Es findet somit ein kontinuierlicher Austausch der gasförmigen Wassermoleküle statt. Deshalb regiert das Messgerät besonders schnell auf Änderungen der Estrichfeuchte.

Der Feuchtesensor 24 ist bevorzugt auf einer Leiterplatte 23 angeordnet, welche auch die Mess- und Kommunikationsschaltung aufnimmt. Die Leiterplatte durchsetzt dazu die beiden Stege 17, welche das Abteil 16 von der Messkammer 15 trennen. Die Leiterplatte 23 wird u.a. durch die Stege 17 mechanisch abgefangen. Die eigentliche Mess-und Kommunikationsschaltung 21, 22 dient zur Erfassung und ggf. Verarbeitung der von entsprechenden Sensoren gelieferten Messwerte, ist in dem Abteil 16 angeordnet und über Leiterbahnen der Leiterplatte mit dem Feuchtesensor 24 oder anderen Sensoren wie z.B. einem beispielhaft dargestellten Temperaturfühler 25 verbunden. Die erfassten Messwerte der Sensoren werden bevorzugt zumindest temporär durch die Mess- und Kommunikationsschaltung gespeichert und können ausgelesen werden. Im skizzierten Beispiel erfolgt das Auslesen durch eine RFID-Datenstrecke, wozu ein handelsüblicher RFID-Chip 21 nebst Antenne Teil der Mess- und Kommunikationsschaltung ist. Alternativ oder zusätzlich zu dem Feuchtesensor kann das Messgerät auch weitere Sensoren, z.B. einen Temperaturfühler 25, Druck- und/oder Kraftsensoren aufweisen. Die entsprechenden Messwerte können in gleicher Weise von der Mess- und Kommunikationsschaltung 20 erfasst und verarbeitet, insbesondere als Funktion der Zeit gespeichert werden.
Oft ist zwischen der Tragschicht 2 und dem Estrich 3 eine Isolierschicht. In diesem Fall kann das Messgerät bevorzugt auf der Isolierschicht angeordnet werden. Wichtig ist nur, dass wenigstens eine der beiden Öffnungen 12, 14 bevorzugt unmittelbar im Estrich angeordnet ist. Besser sind natürlich beide Öffnungen im Estrich angeordnet. Besonders bevorzugt wird das Messgerät derart in den Estrich eingebracht, dass die Öffnung bzw. die Öffnungen zumindest in etwa mittig, bezogen auf die Dicke des Estrichs in diesen eingebracht ist, bzw. sind.

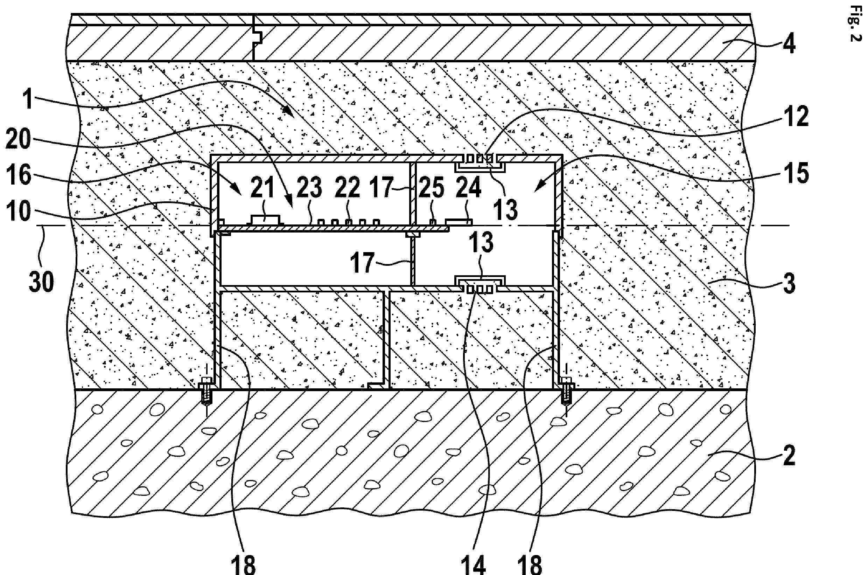

Fig. 2 zeigt ein weiteres Messgerät 1, dass sich nur in Details vom Messgerät nach Fig. 1 unterscheidet. Nur auf diese Details wird eingegangen, im Übrigen gilt die Beschreibung zu Fig. 1 analog. Das Messgerät 1 hat ein Gehäuse 10 mit einer Messkammer 15, welche wie die Messkammer in Fig. 1, zwei wasserdampfdiffusionsoffe Öffnungen 12, 14 aufweist. Die Öffnung 12 ist in der Oberseite des Gehäuses und die Öffnung 14 an der Unterseite des Gehäuses 10 angeordnet, wodurch die Messkammer 15 die beiden Öffnungen 12, 14 wie ein Kanal verbindet. Ziel ist es die beiden Öffnungen zumindest in etwa symmetrisch zur horizontalen Mittelebene 30 des Estrichs anzuordnen, weil dadurch die Luftfeuchte in der Messkammer 15 die Restfeuchte etwa in der Mitte des Estrichs repräsentiert. Deshalb hat das Messgerät 1 als Befestigungsmittel Stützen 18, mit denen das Gehäuse von der als Auflage dienenden Tragschicht 2 aufgesetzt wird. Das Messgerät wird vorzugsweise an der Tragschicht 2 fixiert, z.B. wie gezeigt verschraubt, damit es beim Verlegen des Estrichs 3 nicht verschoben wird oder aufschwimmt. Dazu sind die unteren Enden der Befestigungsmittel 18 im gezeigten Beispiel abgewinkelt, und haben eine Ausnehmung zur Fixierung des Messgeräts mittels wenigstens einer Schraube oder wenigsten einem anderen Verbindungsmittel an der Tragsschicht 2. Alternativ kann zumindest wenigstens eine der Stützen 18 dornartig ausgebildet sein. Diese dornartig ausgebildete Stütze lässt sich dann leicht in plastische Auflagen, wie z.B. eine zwischen dem Estrich und der Tragschicht angeordnete Isolierschicht eindrücken (nicht dargestellt). Besonders bevorzugt ist die Länge der Stütze 18, also des Befestigungsmittels einstellbar, um den Abstand des Gehäuses zur Auflage einstellen zu können, denn dadurch kann die Position der Öffnungen 12, 14 relativ zur Mittelebene 30 eingestellt werden.

Die Stützen 18 dienen somit dazu die Öffnungen 12, 14 zumindest in etwa mittig im Estrich anzuordnen. Mit zumindest in etwa mittig ist gemeint, dass die Differenz der Abstände der Öffnungen 12, 14 zur Mittelebene 30 betragsmäßig vorzugsweise kleiner als der 0,5 fache mittlere Abstand von den Öffnungen 12, 14 zur Mittelebene ist. Besonders bevorzugt ist der Betrag der Differenz kleiner als das 0,25fache (noch besser kleiner als das 0,1 fache) des mittleren Abstands der Öffnungen 12, 14 zu der Mittelebene. Natürlich kann auch das Messgerät 1 nach Fig. 1 auf Stützen wie die Stützen 18 in Fig. 2 angeordnet werden bzw. solche Stützten aufweisen. Die Stützen 18 können in der Höhe einstellbar sein, z.B. als Teleskopstützen ausgebildet sein.

### Bezugszeichenliste

- 1: Messgerät/Restfeuchtesensor
- 2: Tragschicht (hier Stahlbeton)
- 3: Estrich
- 4: Parkett
- 10: Gehäuse
- 12: Öffnung
- 13: Membran
- 14: Öffnung
- 15: Messkammer
- 16: Abteil
- 17: Steg
- 18: Stützen als Beispiel für ein Befestigungsmittel
- 20: Mess-und/oder Kommunikationsschaltung
- 21: RFID-Chip
- 22: Bauelemente
- 23: Leiterplatte
- 24: Feuchtesensor
- 25: weiterer Sensor, z.B. Temperaturfühler
- 30: Mittelebene

## Patentansprüche

1. Estrich mit einem Messgerät (1) zur Bestimmung von Parametern des Estrichs (3) mit zumindest
- einem Gehäuse (10) in dem wenigstens ein Feuchtesensor (24) angeordnet ist, wobei das Gehäuse (10) wenigstens einen Innenraum (16) aufweist,
- wenigstens einer Öffnung (12, 14) des Innenraums (16),
- wenigstens einem Feuchtesensor (24), der in dem Innenraum (16) angeordnet ist,
**dadurch gekennzeichnet, dass**
das Messgerät Befestigungsmittel (18) aufweist, mit denen das Messgerät vor dem Verlegen des Estrichs an einer Tragschicht (2) fixiert wurde, und dass es vollständig in dem Estrich angeordnet ist.

2. Estrich mit einem Messgerät (1) nach Anspruch 1,
**dadurch gekennzeichnet, dass**
die wenigstens eine Öffnung (12,14) durch eine zumindest wasserdampfdurchlässige Membran (13) verschlossen ist.

3. Estrich mit einem Messgerät (1) nach Anspruch 1 oder 2,
**dadurch gekennzeichnet, dass**
der Innenraum als Messkammer (15) einen Kanal aufweist, der wenigstens zwei Öffnungen (12, 14) miteinander verbindet.

4. Estrich mit einem Messgerät (1) nach Anspruch 3 **dadurch gekennzeichnet, dass**
die beiden Öffnungen (12, 14) übereinander angeordnet sind.

5. Estrich mit einem Messgerät (1) nach einem der Ansprüche 1 bis 4
**dadurch gekennzeichnet, dass**
das Gehäuse (10) ein geschlossenes Abteil (16) aufweist, in dem eine Mess-und/oder Kommunikationsschaltung (20) angeordnet ist, welche mit dem Feuchtesensor (24) verbunden ist.

6. Estrich mit einem Messgerät (1) nach Anspruch 5,
**dadurch gekennzeichnet, dass**
das Abteil (16) vergossen ist.

7. Estrich mit einem Messgerät (1) nach einem der vorstehenden Ansprüche,
**dadurch gekennzeichnet, dass**
das Messgerät (1) wenigstens einen Dehnungsmessstreifen und/oder Drucksensor zur Bestimmung von vom Estrich (3) auf das Gehäuse (10) übertragener Kraft aufweist.

8. Estrich mit einem Messgerät (1) nach Anspruch 7,
**dadurch gekennzeichnet, dass**
das Messgerät (1) mit einer Raumüberwachungsanlage verbunden ist, um Messwerte oder daraus abgeleitete Werte an die Raumüberwachungsanlage zu übertragen.

9. Estrich mit einem Messgerät (1) nach einem der vorstehenden Ansprüche
**dadurch gekennzeichnet, dass**
das Messgerät (1) mit einer Steuerung einer Heizungsanlage verbunden ist, um wenigstens einen Messwert von dem Messgerät (1) an die Steuerung zu übertragen, wobei die Steuerung dazu eingerichtet ist die in den Estrich (3) eingebrachte Heizleistung in Abhängigkeit von wenigstens einem der übertragenen Messwerte einzustellen.

10. Estrich mit einem Messgerät (1) nach einem der vorstehenden Ansprüche
**dadurch gekennzeichnet, dass**
dass die Befestigungsmittel (18) längenverstellbar sind, um den Abstand des Messgeräts (1) von der Tragschicht einzustellen.

11. Verfahren zur Kontrolle der Belegreife eines Estrichs (3) nach einem der vorstehenden Ansprüche 1 bis 10, mit zumindest folgenden Schritten:
- Erfassen der Restfeuchte des Estrichs (3) als Funktion der Zeit,
- Bestimmen des Zeitpunkts der Belegreife als den Zeitpunkt, an dem die Restfeuchte einen vorgegebenen Wert unterschreit und/oder konvergiert ist,
**dadurch gekennzeichnet dass**
die Restfeuchte durch wenigstens einen in einem Hohlraum (15) im Estrich (3) angebrachten Luftfeuchtesensor (24) erfasst wird.

12. Verfahren nach Anspruch 11, **dadurch gekennzeichnet, dass**
- die Temperatur des Estrichs (3) als Funktion der Zeit gemessen wird
- die gemessene Temperatur als Funktion der Zeit mit einem gegebenen Temperaturverlauf verglichen wird, und
- anhand des Vergleichs bestimmt wird, ob der Estrich (3) belegreif ist.

13. Verfahren nach Anspruch 11 oder 12
**dadurch gekennzeichnet, dass**
zur Erfassung der Restfeuchte des Estrichs (3) ein Messgerät (1) nach einem der Ansprüche 1 bis 9 verwendet wird.

14. Verfahren insbesondere nach einem der Ansprüche 11 bis 13,
**dadurch gekennzeichnet, dass**
ein Messgerät (1), insbesondere das Messgerät nach einem der Ansprüche 1 bis 10, beim Verlegen des Estrichs (3) vollständig mit Estrich bedeckt wird.

## Claims

1. Screed with a measuring device (1) for determining parameters of the screed (3) with at least
- a housing (10) in which at least one humidity sensor (24) is arranged, wherein the housing (10) comprises at least one interior space (16),
- at least one opening (12, 14) of the inner space (16),
- at least one humidity sensor (24), which is arranged in the interior space (16),
**characterized in that**
the measuring device comprises fixation means (18), by means of which the
measuring device has been fixed to a base layer (2) before laying the screed, and
that it is completely arranged in the screed.

2. Screed with a measuring device (1) according to claim 1,
**characterized in that**
the at least one opening (12, 14) is closed by at least one water vapor permeable membrane (13).

3. Screed with a measuring device (1) according to claim 1 or 2,
**characterized in that**
the interior space as a measuring chamber (15) comprises a channel, which connects at least two openings (12, 14) with each other.

4. Screed with a measuring device (1) according to claim 3,
**characterized in that**
the two openings (12, 14) are arranged one above the other.

5. Screed with a measuring device (1) according to one of claims 1 to 4,
**characterized in that**
the housing (10) comprises a closed compartment (16) in which a measuring and/or communication circuit (20) is arranged which is connected to the moisture sensor (24).

6. Screed with a measuring device (1) according to claim 5,
**characterized in that**
the compartment (16) is potted.

7. Screed with a measuring device (1) according to one of the preceding claims,
**characterized in that**
the measuring device (1) comprises at least one strain gauge and/or pressure sensor for determining a force transmitted by the screed (3) to the housing (10).

8. Screed with a measuring device (1) according to claim 7,
**characterized in that**
the measuring device (1) is connected to a room surveillance system to transfer measured values or values derived from the measured values to the room surveillance system.

9. Screed with a measuring device (1) according to one of the preceding claims,
**characterized in that**
the measuring device (1) is connected to a controller of a heating system, to transmit at least one measured value from the measuring device (1) to the controller, wherein the controller is adapted to adjust the heating power introduced in the screed (3) in dependence of at least one of the transmitted measured values.

10. Screed with a measuring device (1) according to one of the preceding claims,
**characterized in that**
the fixation means (18) are adjustable in length to adjust the distance of the measuring device (1) to the base layer.

11. Method for controlling the overlaying maturity of a screed according to one of the preceding claims 1 to 10, comprising at least the following steps:
- detecting the residual moisture of the screed (3) as a function of time,
- determining the time of the overlaying maturity as the time at which the residual moisture falls below and/or converges a predetermined value,
**characterized in that**
the residual moisture is detected by at least one air humidity sensor (24) arranged in a cavity (15) in the screed (3).

12. Method according to claim 11,
**characterized in that**
- the temperature of the screed (3) is measured as a function of time
- the measured temperature as a function of time is compared with a given temperature curve,
- it is determined based on the comparison, whether the screed (3) is ready for applying a subsequent layer.

13. Method according to claim 11 or 12,
**characterized in that**
a measuring device (1) according to one of claims 1 to 9 is used for detecting the residual moisture of the screed (3).

14. Method in particular according to one of claims 11 to 13,
**characterized in that**
a measuring device (1), in particular the measuring device according to one of claims 1 to 10, is completely covered by screed when laying the screed (3).

## Revendications

1. Chape de béton avec un appareil de mesure (1) pour la détermination de paramètres de la chape de béton (3), avec au moins
- un boîtier (10) dans lequel est disposé au moins un capteur d'humidité (24), lequel boîtier (10) présente au moins un espace intérieur (16),
- au moins une ouverture (12, 14) de l'espace intérieur (16),
- au moins un capteur d'humidité (24) qui est disposé dans l'espace intérieur (16),
**caractérisé en ce que**
l'appareil de mesure présente des moyens de fixation (18) avec lesquels il est fixé sur une couche de support (2) avant l'application de la chape de béton, et
**en ce qu'**il est entièrement disposé dans la chape de béton.

2. Chape de béton avec un appareil de mesure (1) selon la revendication 1, **caractérisé en ce que** l'au moins une ouverture (12, 14) est fermée par une membrane (13) perméable au moins à la vapeur d'eau.

3. Chape de béton avec un appareil de mesure (1) selon la revendication 1 ou 2, **caractérisé en ce que** l'espace intérieur présente comme chambre de mesure (15) un canal qui relie entre elles au moins deux ouvertures (12, 14).

4. Chape de béton avec un appareil de mesure (1) selon la revendication 3, **caractérisé en ce que** les deux ouvertures (12, 14) sont disposées l'une au-dessus de l'autre.

5. Chape de béton avec un appareil de mesure (1) selon l'une des revendications 1 à 4, **caractérisé en ce que** le boîtier (10) présente un compartiment fermé (16) dans lequel est disposé un circuit de mesure et/ou de communication (20) qui est relié au capteur d'humidité (24).

6. Chape de béton avec un appareil de mesure (1) selon la revendication 5, **caractérisé en ce que** le compartiment (16) est rempli avec une masse coulée.

7. Chape de béton avec un appareil de mesure (1) selon l'une des revendications précédentes, **caractérisé en ce que** l'appareil de mesure (1) présente au moins une jauge extensométrique et/ou un capteur de pression pour déterminer la force transmise par la chape de béton (3) au boîtier (10).

8. Chape de béton avec un appareil de mesure (1) selon la revendication 7, **caractérisé en ce que** l'appareil de mesure (1) est relié à un installation de surveillance de la pièce afin de transmettre des valeurs de mesure ou des valeurs dérivées de celles-ci à l'installation de surveillance de la pièce.

9. Chape de béton avec un appareil de mesure (1) selon l'une des revendications précédentes, **caractérisé en ce que** l'appareil de mesure (1) est relié à une commande d'une installation de chauffage, afin de transmettre au moins une valeur de mesure de l'appareil de mesure (1) à la commande, laquelle commande est aménagée de façon à ajuster la puissance de chauffage transmise dans la chape de béton (3) en fonction d'au moins une des valeurs de mesure transmises.

10. Chape de béton avec un appareil de mesure (1) selon l'une des revendications précédentes, **caractérisé en ce que** les moyens de fixation (18) sont réglables en longueur afin d'ajuster la distance entre l'appareil de mesure (1) et la couche de support.

11. Procédé pour contrôler le moment où une chape de béton (3) selon l'une des revendications 1 à 10 est prêt à être recouvert, comprenant au moins les étapes suivantes :
- détection de l'humidité résiduelle de la chape de béton (3) en fonction du temps,
- détermination du moment où la chape de béton peut être recouvert comme le moment où l'humidité résiduelle passe en dessous d'une valeur prédéterminée et/ou converge avec celle-ci,
**caractérisé en ce que** l'humidité résiduelle est détectée par au moins un capteur d'humidité de l'air (24) disposé dans une cavité (15) dans la chape de béton (3).

12. Procédé selon la revendication 11, **caractérisé en ce que**
- la température de la chape de béton (3) est mesurée en fonction du temps ;
- la température mesurée en fonction du temps est comparée à une évolution de la température donnée et
- déterminer si la chape de béton (3) est prêt à être recouvert a partir de la comparaison.

13. Procédé selon la revendication 11 ou 12, **caractérisé en ce qu'**un appareil de mesure (1) selon les revendications 1 à 9 est utilisé pour détecter l'humidité résiduelle de la chape de béton (3).

14. Procédé, en particulier selon l'une des revendications 11 à 13, **caractérisé en ce qu'**un appareil de mesure (1), en particulier l'appareil de mesure selon l'une des revendications 1 à 10, est entièrement recouvert de chape de béton lors de la pose de la chape de béton (3).
